# EUROPEAN PATENT SPECIFICATION

(11) **EP 3 079 819 B1**
(45) Date of publication and mention of the grant of the patent: **14.08.2019**
(21) Application number: 14799151.7
(22) Date of filing: 14.11.2014
(51) Int. Cl.: B01L 3/00

(54) **SAMPLE STORAGE AND EXTRACTION DEVICE FOR FLOW THROUGH ELUTION OF ANALYTES**
PROBENLAGERUNGS- UND EXTRAKTIONSVORRICHTUNG FÜR DURCHFLUSSELUIERUNG VON ANALYTEN
DISPOSITIF DE STOCKAGE ET D'EXTRACTION D'ÉCHANTILLONS POUR ÉLUTION À ÉCOULEMENT CONTINU D'ANALYTES

(30) Priority: 13.12.2013 US 201314105204
(43) Date of publication of application: 19.10.2016
(73) Proprietor: General Electric Company, Schenectady, NY 12345 (US)
(72) Inventor: GRIFFIN, Weston, Blaine, Niskayuna, New York 12309 (US); FINEHOUT, Erin, Jean, Niskayuna, New York 12309 (US); MAO, Ying, Niskayuna, New York 12309 (US); LENIGK, Ralf, Niskayuna, New York 12309 (US); ERNO, Daniel, Jason, Niskayuna, New York 12309 (US)
(74) Representative: Cavill, Ross David
(86) International application number: PCT/EP2014/074673
(87) International publication number: WO 2015/086261

(56) References cited:
- EP-A1- 2 330 402
- WO-A1-02/33380
- WO-A1-2006/081021
- US-A1- 2004 259 168
- US-A1- 2009 117 660
- US-A1- 2012 018 629

## Description

### BACKGROUND

The invention relates to storage of biological samples, and more particularly to devices configured to store and analyze the biological samples.

The collection of biological samples (such as blood) and extracting DNA for genetic analysis from the sample have been widely used by the forensics and medical community for identification purposes such as, but not limited to, paternity testing, genetic diagnostic testing in new born screening programs, genetic typing for predisposition to a particular disease, and genetic characterization for drug susceptibility. Typically, collected biological samples are stored in a dried state on an absorbent material. By way of example, dried blood spots are commonly stored on sample substrates. In a typical workflow for sample collection and preparation, the sample is applied to the substrate, the sample is allowed to dry, and then the sample is transported to the analysis site. The sample must be dried before transport because when a wet sample is transported, a portion of the sample may inadvertently transfer to one or more surfaces that come in contact with the sample.

One approach of sample collection and isolation for analysis of the sample includes cutting out a portion of a sample substrate card having a dry sample. The cut portion of the sample substrate is placed in a vial or a well. An extraction fluid is added to the vial. The vial is shaken or vortexed for a set period of time. However, this approach of cutting the portion of the sample substrate experiences risks of losing samples from the cut portions, sample contamination, and contamination from the cutting device, e.g., blade.

Alternatively, in another approach, a portion of the sample substrate on which the sample is to be collected may be pre-cut and positioned in a device that allows the fluid to flow through the device to extract analytes. The sample may be disposed on after the sample substrate is cut, and the sample is extracted from cut portion using one or more of vortexing, shaking, and flow-through. Although pre-cutting the sample substrate may address risks associated with cutting the sample substrate after disposing the sample.. However, pre-cutting approach has limited application of use and does not allow a user to analyze the sample multiple number of times.

Yet another approach for sample collection and isolation includes placing the sample substrate on a hard surface and pressing down a portion of the sample substrate with a sharp-edge having a determined shape. For example, the sample substrate may be pressed down with a circular knife-edge. The sharp edge is such that the sharp edge presses against the sample substrate but does not cut through the sample substrate. Extraction buffers are then passed over the surface of the portion of the sample substrate that is isolated by the knife-edge. This method avoids cutting, but does not ensure that the fluid extracts sample from the full depth of the sample substrate (e.g. only the analytes at the surface may be extracted). It also does not provide a way to remove the fluid from the isolation zone of the card before removing the knife-edge. The fluid remaining in the isolation zone may result in fluid wicking into the surrounding area after the knife-edge is removed. This wicking may damage the remaining sample making re-sampling from another position on the card difficult or impossible.

Existing workflows for the sample substrate cutting and extraction, poses several problems when facing the challenge of automation. The primary problems arise from the cutting step. The small cut discs are highly prone to the effects of static electricity or even a light breeze. There are numerous reports of cut discs being lost during the cutting step or during transport of the cut discs. Moreover, automated processes involving existing approaches suffer the problems and risks associated with wicking of the areas outside the isolation zone, and accurate and consistent amount of sample collection in the desirable area of the sample substrate.

Another approach for sample recovery from a substrate is to use a compressive force to create a seal around the area of interest. Extraction fluid may then be allowed to flow perpendicular to the substrate, through the area of interest. This approach has been used in automated systems, usually using metal or ceramic parts to form the compression seal. In such systems, the same sealing parts are used for multiple samples. The re-use of parts may not be possible in fields where contamination may be detected at very low levels. For example, when extracting DNA/RNA from a biological sample and then performing amplification. In these cases a single copy of DNA/RNA that is carried over may be detected and give a false positive. Prior sample and/or extraction devices are disclosed in US2012/018629, EP 2330402, WO02/33380, US, 2009/117660, and US2004/259168.

### BRIEF DESCRIPTION

In one embodiment, a sample storage and extraction device is provided as claimed in claim 1. The sample storage and extraction device includes a substrate frame and a substrate cover. The substrate frame includes a substrate region configured to receive a sample substrate. The sample storage and extraction device further includes a compression assembly configured to provide an isolation zone in a portion of the sample substrate. Moreover, the sample storage and extraction device includes a fluidic channel configured to flow elution fluid to the isolation zone.

In another embodiment, a method of use of a sample storage and extraction device is provided as claimed in claim 6. The method includes providing a sample storage and extraction device, operatively coupling the sample storage and extraction device to an external device, and providing an isolation zone in a portion of a sample substrate by applying a force on the sample storage and extraction device in a determined direction. The method further includes flowing washing and elution fluid through or across the isolation zone in the portion of the sample substrate, releasing the compression force; and analyzing the portion of the sample in the isolation zone.

In yet another embodiment, a disposable sample storage system is provided as claimed in claim 13.

### DRAWINGS

These and other elements and aspects of aspects of the present specification will become better understood when the following detailed description is read with reference to the accompanying drawings in which like characters represent like parts throughout the drawings, wherein:
FIG. 1 is an exploded view of an example sample storage and extraction device where a substrate cover is configured to slide over a substrate frame, in accordance with aspects of the present specification;
FIG. 2 is a perspective view of the sample storage and extraction device of FIG. 1 in an exposed state of a sample substrate, in accordance with aspects of the present specification;
FIG. 3 is a perspective view of the sample storage and extraction device of FIG. 1 where the sample substrate having the sample is covered to facilitate sample handling, transport and sample elution, in accordance with aspects of the present specification;
FIG. 4 is a perspective view of an example sample storage and extraction device having a substrate frame and substrate cover, where the sample storage and extraction device includes a foldable structure, in accordance with aspects of the present specification;
FIG. 5 is a perspective view of the sample storage and extraction device of FIG. 4 without a laminate layer disposed on the substrate cover to illustrate features disposed underneath the laminate layer, in accordance with aspects of the present specification;
FIG. 6 is a side view of an example sample storage and extraction device in a folded state, in accordance with aspects of the present specification;
FIG. 7 is a perspective view of an example sample storage and extraction device employing a living hinge, where the living hinge includes a plurality of portions that together provide reversible folding and unfolding of a substrate frame and a substrate cover, in accordance with aspects of the present specification;
FIG. 8 is a flow chart of an example method of using the sample storage and extraction device, in accordance with aspects of the present specification;
FIG. 9 is a side view of an example sample storage and extraction device operatively coupled to an external device, where a compression member of a compression assembly is disposed in a substrate frame of the sample storage and extraction device, and where a secondary member of the compression assembly is disposed in the external device, in accordance with aspects of the present specification;
FIG. 10 is a perspective view of a system having an example sample storage and extraction device and an external device, in accordance with aspects of the present specification;
FIG. 11 is a perspective view of a portion of the system of FIG. 10, in accordance with aspects of the present specification;
FIG. 12 is a perspective view of an example sample storage and extraction device configured to be coupled to an external device, in accordance with aspects of the present specification;
FIG. 13 is a perspective view of a portion of an example sample storage and extraction device employing a compression assembly, in accordance with aspects of the present specification;
FIG. 14 is an example flow diagram of a method of using a sample storage and extraction device of the present disclosure, in accordance with aspects of the present specification;
FIG. 15 is a perspective view of an example arrangement before and after a fluidic communication between an external device and a sample storage and extraction device is established, in accordance with aspects of the present specification;
FIG. 16 is a perspective view of an example sample storage and extraction device employing a heating element, in accordance with aspects of the present specification; and
FIG. 17 is a schematic representation of an example flow diagram for achieving a desirable elution solution temperature, in accordance with aspects of the present specification.

### DETAILED DESCRIPTION

Aspects of the present specification relate to single-use disposable sample storage and extraction devices. The sample storage and extraction devices are configured to house at least a portion of a sample substrate. In some embodiments, a sample storage and extraction device may be coupled to a sample collection device to receive at least a portion of a biological sample from the sample collection device on the sample substrate of the sample storage and extraction device. Further, the sample storage and extraction device may be configured to store the received sample for further processing and analyzing. In one embodiment, the sample storage and extraction device may be configured to facilitate flow of liquids through desirable areas of the sample substrate having the biological sample to facilitate washing and extraction of the sample from the sample substrate. In certain embodiments, the sample storage and extraction devices may be integrated with temperature control devices to facilitate control of heating of the elution solution to a desirable temperature. Alternatively or additionally, in some embodiments, the sample storage and extraction devices may be coupled to another external device for sample elution and processing. In a non-limiting example, the external device may include a fluidic device.

The sample storage and extraction device of the present disclosure may be employed in assemblies or systems that are configured to perform one or more of collection, transfer, storage, and analysis of one or more biological samples in a controlled manner. By way of example, the sample storage and extraction device may be used in systems used to collect degradable biologically sourced analytes such as nucleic acids, proteins, and respective fragments thereof. It should be noted that the terms "sample" and "biological sample" may be used interchangeably throughout the application. Non-limiting examples of the biological sample may include saliva, blood, serum, lymph fluids, buccal cells, mucosal cells, cerebrospinal fluid, semen, feces, plasma, urine, a suspension of cells, or a suspension of cells and viruses. In addition, the biological samples may include samples from flora and fauna. In a non-limiting example, the biological samples may include plant or fungal samples for the study of population genetics. In one example, the assembly may be used for storage and analysis of biological samples for purposes, such as but not limited to, collection of buccal cell samples for criminal databases, collection of crime scene samples (i.e., rehydrated blood, semen, saliva and liquid samples of the same), collection of sexual assault samples, collection of buccal samples for population genetics or pharmacogenomics studies, collection of nasal samples for respiratory infection diagnosis, collection of bacterial or parasite samples from food sources, collection of blood from meat at slaughterhouse for meat traceability, or collection of biological samples from animals for veterinary diagnostics. It should be noted that at the time of collection, the biological samples may or may not exist in a biological body from where the sample originated. By way of example, the biological sample may include a blood sample splattered on a floor of a crime scene.

In certain embodiments, a sample storage and extraction device is a single-use disposable device that is configured to hold the sample substrate to facilitate flow of the solutions through desirable areas of the sample substrate disposed in the sample storage and extraction device. In some embodiments, the sample storage and extraction device may be in operative association with a fluidic device for sample elution and processing via a connected instrument. In certain embodiments, the sample storage and extraction device may be part of the system that also includes a sample collection device. In one embodiment, the sample collection device may be configured to receive at least one sample collection member. The sample collection member is configured to collect a biological sample. The sample substrate of the sample storage and extraction device may be configured to receive and store at least a portion of the sample from the sample collection device. In some embodiments, one or more parts of the single-use disposable sample storage and extraction device may be configured for one time use to reduce or prevent contamination or spreading of infection via the sample storage and extraction device. In certain embodiments, the sample storage and extraction device may be configured for reliable and reproducible collection, transfer and storage of biological samples. In certain embodiments, a percentage of the biological sample transferred from the sample collection device to a sample storage and extraction device may be reproducible.

In some embodiments, the sample substrate may include at least one stabilizing reagent that preserves at least one biological sample analyte for transport or storage. Non-limiting examples of suitable reagents for the storage media may include one or more of a weak base, a chelating agent, and, optionally, uric acid or a urate salt or simply the addition of a chaotropic salt, alone or in combination with a surfactant. In one embodiment, the sample substrate may have a visual delineation disposed around a transfer area of the sample substrate such that, if the sample storage and extraction device is removed from the assembly or system, an operator can know where the material was deposited without reference to the assembly or system.

In some embodiments, the design of the sample storage and extraction device facilitates safe collection, transfer and storage of the sample, while preventing any undesirable contact of the user with the sample while transferring of the sample from the sample collection device to the sample storage and extraction device, or while storage of the sample. By way of example, in some embodiments, the sample storage and extraction device may include a cover to enclose the sample once the sample is transferred from the sample collection device to the sample storage and extraction device. The enclosed biological sample may be shipped to a desirable destination (e.g., a lab) for further analysis. In one embodiment, the enclosure may be allowed to stand for a desirable period of time to allow the sample substrate to dry prior to shipping the enclosure. In other embodiments, the enclosure may be disposed in a case (e.g., a foil pouch) along with a desiccant prior to shipping. In one embodiment, the substrate cover of the device may protect the sample from undesirable contact during drying.

In certain embodiments, the sample storage and extraction device may facilitate a reagent fluid to flow through desirable areas of the sample substrate to facilitate washing or analyte elution from the sample. The sample may be a wet or a dry sample. For example, the sample may be a dried blood sample. In some embodiments, the reagent fluid may be passed through the desirable areas of the sample substrate by isolating the desirable areas from the rest of the sample substrate. The isolation may be such that major portion of the reagent fluid may be passed through only the desirable areas of the sample substrate. In one embodiment, a reagent fluid may be passed through an isolation zone of the sample substrate to extract at least a portion of the biological sample from the sample substrate. In one example, the reagent fluid may pass through the isolated portion in a direction perpendicular to the plane of the sample substrate. Isolating an area of the sample substrate facilitates one or more tests to be applied to the portion of the sample substrate that is isolated by compression. Effectively isolating a portion of the sample substrate facilitates a consistent quantity of sample being tested. For example, even if the amount of blood collected on the sample substrate varies, the sample storage and extraction device is configured to extract and/or analyze the sample from the isolation zone for a plurality of times to facilitate consistent quantity of sample being tested. As will be appreciated, for commercially available sample substrates that are susceptible to wicking out uniformly, analyzing a determined area of the sample substrate is equivalent to analyzing the same volume of the original sample material. Improving the automation solutions available for the handling of the sample substrate enables the increased use of the sample storage and extraction devices in fields such as the pharmaceutical industry which require high-throughput analysis. Non-limiting examples of the sample substrate may include a porous sample substrate, Whatman FTA™ card, cellulose card, or combinations thereof.

In certain embodiments, a sample storage and extraction device may include two portions, a substrate frame and a substrate cover. The two portions of the sample storage and extraction device may include a sample substrate, a compression assembly, fluidic channels, flexures, optionally, a heating element and one or more membranes. In some embodiments, the sample storage and extraction device may include a multilayer structure. The multilayer structure may be configured to house a sample substrate and one or more fluidic channels. The fluidic channels may be configured to provide fluidic connection between the sample substrate and one or more external devices. The substrate frame and substrate cover may include features to facilitate fluid flow through a region of interest (e.g., at the center of the applied sample area) disposed on the sample substrate

In some embodiments, the sample storage and extraction device may include a compression assembly having a compression member and a secondary member. The compression assembly is configured to isolate a portion or zone of the sample substrate while allowing fluidic access to the isolation zone. In one embodiment, the compression assembly may be configured to isolate a sample region, where the sample region is a portion of the sample substrate having the sample. The isolation zone may include at least a portion of the sample disposed on the sample substrate. In one example, the substrate frame may have an opening corresponding to the isolation zone defined by the compression assembly. The compression member of the compression assembly in conjunction with the secondary member may be configured to provide isolation to the isolation zone of the sample substrate with respect to fluids that may be flowed through the sample substrate for processing of the sample disposed on the sample substrate. The portion of the sample in the isolation zone may be processed and analyzed while minimizing wicking of fluids into the areas of the sample substrate disposed outside the isolation zone.

Further, the sample storage and extraction device may include a fluid inlet having access to the isolation zone. In one example, the fluid inlet may have access to the isolation zone when the compression assembly is pressed against the sample substrate. The compression assembly may be pressed against the sample substrate by disposing the compression member and the secondary member on opposite sides of the sample substrate and pressing the compression and secondary members in opposite directions towards each other to define the isolation zone. Further, the sample storage and extraction device may include a fluid outlet to receive outgoing fluid from the isolation zone. The fluid in the sample storage and extraction device may flow towards or away from the isolation zone. By way of example, the fluid may be directed towards the isolation zone for sample extraction. Further, the fluid may be directed away from the isolation zone after the sample extraction. The fluid outlet may be provided through the opening in the substrate frame corresponding to the isolation zone, at least when the compression assembly is pressed against the porous sample substrate. The fluid inlet and outlet may allow the device to be connected to an external device that provides the fluids and/or collects the extracted sample.

In certain embodiments, the sample storage and extraction device may include a flexure configured to be disposed around a sample region. Further, the flexure may be configured to distribute an applied force to form the isolation zone. In some embodiments, the sample storage and extraction device may include a dual flexure design. In operation, the dual flexure design facilitates application of uniform pressure around the isolation area. Application of the uniform pressure around the isolation area enhances the sealing of the isolation area. In addition to facilitating uniform pressure around the isolation area, the flexures also facilitate spanning of the compression member across the air gap above the isolation area while using relatively lower values of applied forces.

In some embodiments, the substrate cover of the sample storage and extraction device may include a cover, such as, but not limited to, a folding cover or a sliding cover, to protect the sample substrate from accidental contact or contamination during handling and transport. Moreover, the cover enables the elution fluid to flow through the sample substrate when the sample storage and extraction device is operatively coupled to a downstream device or instrument.

In certain embodiments, to extract the sample from the sample substrate, the elution fluid or extraction buffer may flow through the sample substrate using inlet and outlet connections provided in the sample storage and extraction device. In some of these embodiments, sealing features of the compression member may be in direct contact with the sample substrate to facilitate the fluid flow through the isolation zone of the applied sample area. In one example, the elution fluid is heated up to desired temperature before flowing the fluid through the sample substrate. An applied external force (from an external instrument) pushes the sealing features and thus the fluid channels against the sample substrate for eluate flow through the region of interest and the external force also seals the inlet/outlet channels between the sample storage and extraction device and the connections to the instrument. In one embodiment, the applied external force pushes against the flexures of the sample storage and extraction device, the flexures in turn cause the sealing features of the compression ring to push against the sample substrate thereby creating a isolation zone. In addition to providing the isolation zone, the sample storage and extraction device may be configured to transfer at least a portion of the sample from the sample collection device, and transport the sample to a sample analysis instrument. In some embodiments, a washing buffer may be allowed to flow through the isolation zone followed by the extraction buffer. By way of example, the washing buffer may be allowed to flow through the isolation zone to remove contaminants from the sample.

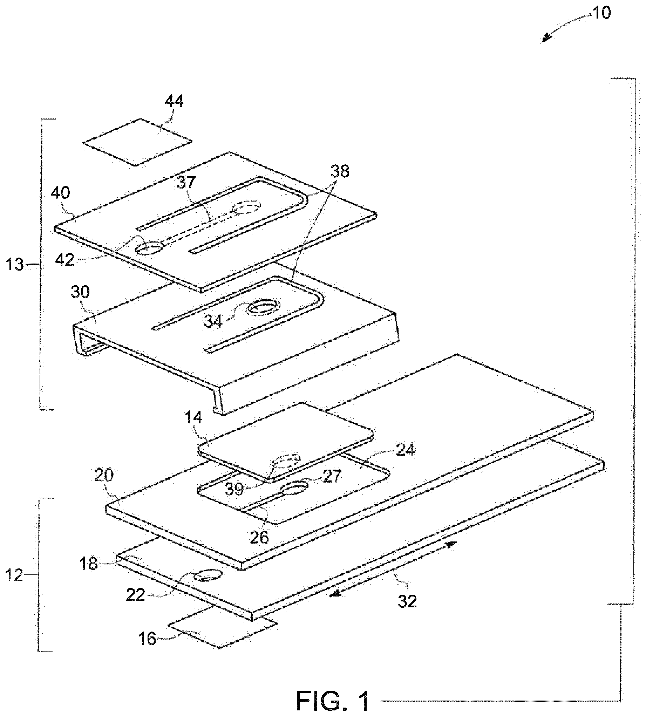

FIG. 1 illustrates an exploded view of a design of an example sample storage and extraction device 10. The sample storage and extraction device 10 includes a substrate frame 12 and a substrate cover 13. At least a portion of a sample substrate 14 may be disposed on the substrate frame 12. In certain embodiments, the substrate frame 12 may include a multilayer structure. The multilayer structure of the substrate frame 12 may include a protective layer 16. In one example, the protective layer 16 may be configured to protect the interface port 22 from contamination. Further, the substrate frame 12 may include protective layer 16, a base layer 18, and a holder base 20. The protective layer 16 is configured to be perforated or disintegrated to allow a fluidic device (not shown) to access an interface port 22 of the base layer 18. The interface port 22 may be used as a fluid inlet and/or outlet port for a reagent fluid. The holder base 20 may include a substrate indentation 24 configured to receive the sample substrate 14. The holder base 20 may further include a first fluidic channel 26 such that a fluid passing to/from the interface port 22 from/to the sample substrate 14 only comes in contact with the sample substrate 14 within an isolation zone (not shown) disposed at an entrance of the fluidic channel 26. In one example, the fluidic channel 26 may be a microfluidic channel. The fluidic channel 26 facilitates fluidic communication between a fluid source (not shown) with the sample substrate 14. The fluid source may be external to the sample storage and extraction device 10.

The substrate cover 13 may include a cover base 30, a cover layer 40, and a membrane 44. The holder base 20 and the cover base 30 together form a receptacle configured to house the sample substrate 14, while protecting the sample substrate 14 from any undesirable accidental contact with the user, or undesirable exposure to the ambient fluids. Accordingly, the sample disposed on the sample substrate 14 is protected from undesirable contacts with the user or undesirable exposure to the ambient fluids. In the illustrated embodiment, the cover base 30 is configured to slide over the holder base 20 in directions generally represented by the arrow 32.

To minimize wicking of the elution fluid, the holder base 20 or cover 30, or both, may include a compression assembly. In some embodiments, the compression assembly may be disposed on a flexure. In particular, in some embodiments, a compression member of the compression assembly may be disposed on the flexure and the secondary member of the compression assembly may not be disposed on the flexure.

A determined amount of external force may be applied to seal the compression member and the secondary member against the sample substrate for flow through/across elution of the region of interest. Non-limiting examples of the compression member 34 may include a raised annular boss or compression ring on a surface of the substrate cover 13 that is in contact with the sample substrate 14 to seal at least a portion of the sample substrate 14 when force is applied to a flexure 38 to effectively seal the channels around the isolation zone of the sample substrate 14. The compression member 34 may be disposed in a position so as to be aligned with a through hole 27 present on the holder base 20. The substrate cover 13 may further include a flexure 38. In the illustrated embodiment, the fluidic channel 26 in the substrate indentation 24 may traverse from the inlet/outlet to the center of the flexure 38. The flexure 38 may be configured to provide uniform pressure around the isolation zone. In operation, an external force applied to the sample storage and extraction device 10 may be transferred to the flexure 38. The force on the flexure 38 may cause the compression member 34 to push against the sample substrate 14 to provide the isolation zone on the sample substrate 14. The isolation zone may be sealed and isolated from the rest of the sample substrate 14 such that there is no fluidic communication between the isolation zone and areas of the sample substrate 14 disposed outside the isolation zone. The sealing area may correspond to an area 39 of the substrate 14 that comes in contact with the compression member 34. Accordingly, when a sealing feature of the compression member 34 is in the form of a ring, the sealing area may be in the form of a circle having similar area as that of the face or contact surface of sealing feature. It should be noted that the size and shape of the compression member 34 may be varied depending on a size and shape desirable for the isolation zone based on a given application or use of the device. In certain embodiments, the first and second parts of the compression member 34 may be pressed against the sample substrate 14 to provide an isolation zone. In particular, the first and second parts of the compression member 34 may be pressed against opposite sides of the sample substrate 14 to provide an isolation zone. In one embodiment, the first part may have access to fluid inlet, and the second part may have access to fluid outlet.

The sample storage and extraction device 10 may further include a cover layer 40. The cover layer 40 may include a second fluidic channel 37 corresponding to the first fluidic channel 26. In case of flow through elution of the sample disposed in the sample substrate 14, the fluid may be pumped through the fluidic channel 37 and the sample substrate 14, and pass via the first fluidic channel 26. In case of one-sided fluid access, the first fluidic channel 26 may include a hydrophobic membrane to allow air to pass but not other fluids. The elution fluid may be pumped to and from the sample substrate 14 through fluidic channel 37 to enhance elution. The cover layer 40 may further include an interface port 42 corresponding to the interface port 22 of the base layer 18. Both ports 22 and 42 disposed on the holder base 20 and substrate cover 13, respectively, are sealed with the protective layer 16 and 44, respectively, to minimize contamination during handling of the holder.

Various layers of the sample storage and extraction device 10 may be made of plastic. In some embodiments, some or all of the components of the sample storage and extraction device 10 may be disposable in nature. By way of example, the compression member of the sample storage and extraction device 10 may be disposable in nature. In some embodiments, the sample substrate frame 10 may be made using additive manufacturing. Advantageously, additive manufacturing techniques may enable the device to take the form of a single structure for each key component (e.g., substrate frame) rather than multilayer components. In one example, the sample storage and extraction device may be made using low cost and high throughput methods, such as, but not limited to, injection molding.

In certain embodiments, the sample storage and extraction device 10 having the sample substrate 14 may be operatively coupled to a sample collection device (not shown). The sample collection device may include a sample collection member, such as, but not limited to a swab. The sample storage and extraction device 10 may be configured to facilitate consistent sample application to the sample substrate 14 by a trained or untrained user. In one embodiment, after the transfer of the biological sample, at least a portion of the sample collection device may be discarded. The substrate cover 13 is configured to protect the sample substrate 14 from accidental and/or undesirable contact of the sample during handling of the sample storage and extraction device 10. In operation, operatively coupling the sample substrate 14 to the sample collection device automatically slides the substrate cover 13 and exposes the sample substrate 14. Upon removal of the sample substrate frame 10 by the user, the substrate cover 13 is repositioned over the sample substrate for handling protection.

In the illustrated embodiment, the cover layer 40 includes features to enable fluid flow through a region of interest (e.g., the isolation zone). However, it may be noted that the features configured to enable the fluid flow may be present in the cover base 30, the cover layer 40, or both. In one embodiment, fluidic channels may be created using one or more layers of plastic sample substrate.

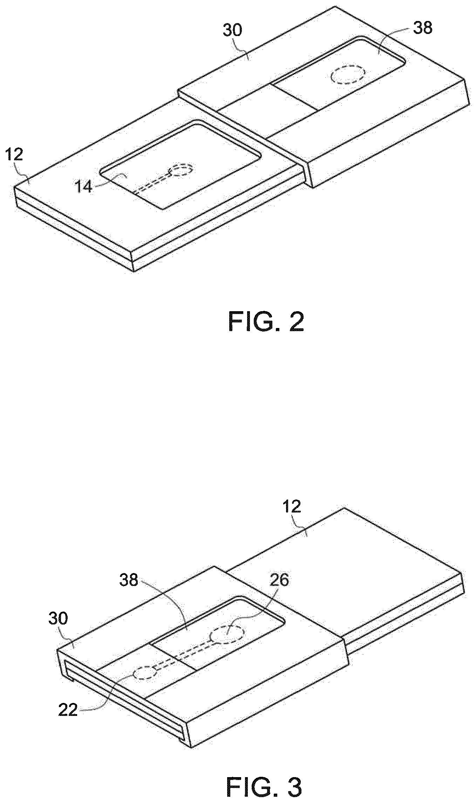

FIGS. 2 and 3 illustrate two different positions of the substrate cover 13 (see FIG. 1) on the holder base 12 (see FIG. 1). In the illustrated embodiment of FIG. 2, the holder base 12 with the sample substrate 14 (see FIG. 1) is exposed for sample transfer. Whereas, in the illustrated embodiment of FIG. 3, the holder base 12 with the sample substrate 14 is covered for handling, transport and sample elution. In one embodiment, when the sample storage and extraction device 10 is operatively coupled to the analysis unit, the substrate cover 13 may be used to cover the sample disposed in the isolation zone of the substrate 14. By way of example, when a portion of the sample storage and extraction device 10 is operatively coupled to an external device (not shown) the substrate cover 13 may be used to cover the sample during analysis. In another embodiment, after analysis, when required, the sliding substrate cover 13 may be moved to expose the sample. It should be noted that the external device may be any device or instrument that is external to the sample storage and extraction device. Non-limiting examples of the external device may include a fluidic device (e.g., a microfluidic device), an analysis instrument, a device configured to mate with a portion of the sample storage and extraction device 10, or combinations thereof. In a particular example, the external device may be a microfluidic device.

Further, insertion of the sample storage and extraction device 10 in a fluidic device may perforate the protective layers 16 and 44 (see FIG. 1), thereby facilitating fluid to be pumped from the fluidic device to the sample substrate 14 via the fluidic channel 26 and 37 (see FIG. 1). In one example, where the external device is a fluidic device, once the sample storage and extraction device 10 is operatively coupled to the external device, an actuator within the external device may apply a force to the flexures 38 to seal compression members 34 against the sample substrate 14.

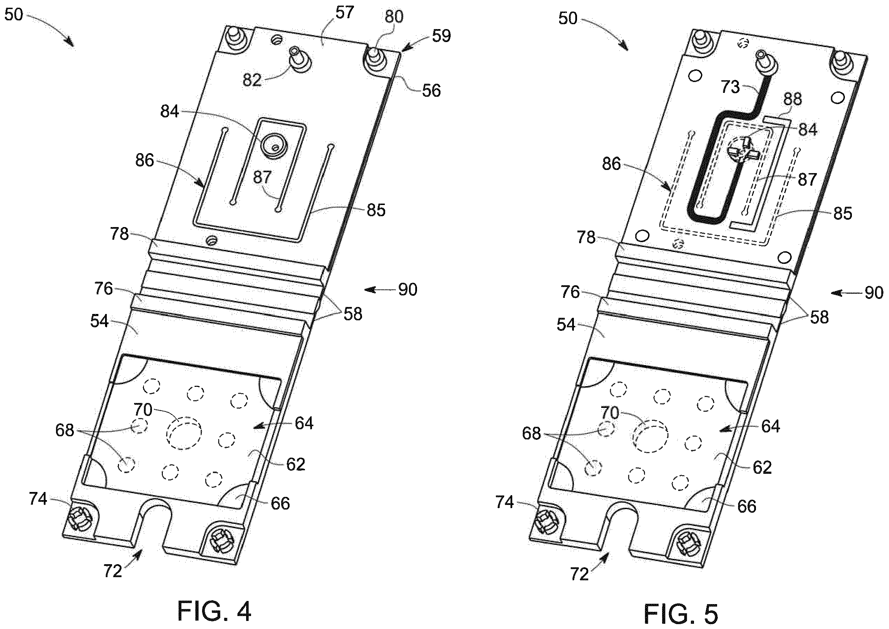

FIGS. 4-5 illustrate an alternative embodiment of the sample storage and extraction device 10 of FIGS. 1-3 that has a slidable structure. In the illustrated embodiment, the sample storage and extraction device 50 includes a foldable structure. In particular, the sample storage and extraction device 50 includes a substrate frame 54 and a substrate cover 56 that may be folded onto each other. The substrate frame 54 and substrate cover 56 may be coupled using a living hinge 58 or other fasteners that may allow the substrate frame 54 and substrate cover 56 to fold and unfold reversibly. In particular, the living hinge 58 or other fasteners may be such that the substrate frame 54 and substrate cover 56 may be folded and unfolded more than once as desirable. In the illustrated embodiment, the substrate cover 56 may include a laminate layer 57 disposed on at least a portion of the substrate cover 56. The laminate layer 57 may be configured to provide protection to one or more components disposed on the substrate cover. It may be noted that the laminate layer 57 in conjunction with the substrate cover 56 may define a fluidic channel 73.

In one embodiment, the living hinge 58 may include one or more thin flexible hinges or flexures. In one example, the living hinge 58 may be made from the same material as the substrate frame 54 and substrate cover 56. Non-limiting examples of the material for the substrate frame 54 and substrate cover 56 may include, plastic, e.g., polypropylene. The living hinge 58 may be configured to limit the relative rotational motion between the substrate frame 54 and substrate cover 56. Further, the living hinge 58 may be configured to provide fatigue resistance to the assembly 50. In one example, the living hinge 58 may be injection molded. In another example, the entire sample storage and extraction device 50 including the sub-components may be injection molded. Upon application of a determined amount of force, the living hinge 58 may be flexed such that a load is applied to the sample collection member to assist in sample transfer from the sample collection member to the substrate frame 54.

The substrate frame 54 may include a sample substrate region 62 configured to receive the sample substrate 64. The sample substrate region 62 may include holder areas 66 to mechanically couple the sample substrate 64 to the sample substrate region 62. In one embodiment, the holder areas 66 may include one or more fasteners to couple the sample substrate 64 to the substrate frame 54. In another embodiment, the holder areas 66 provide a location for adhesive to be applied to couple the samples substrate 64 to the substrate frame 54. The sample substrate region 62 may include gap standoffs 68 and an exit port clearance 70 for washing and elution. In one embodiment, the exit port clearance 70 may serve as a clearance hole for a mating component, where the mating surface may serve as the exit port. The exit port clearance 70 may correspond to a position of a compression member 84 of a compression assembly. In the illustrated embodiment, the compression member 84 is disposed in the substrate cover 56 or the laminate layer 57. A secondary member of the compression assembly is not shown in the illustrated embodiment.

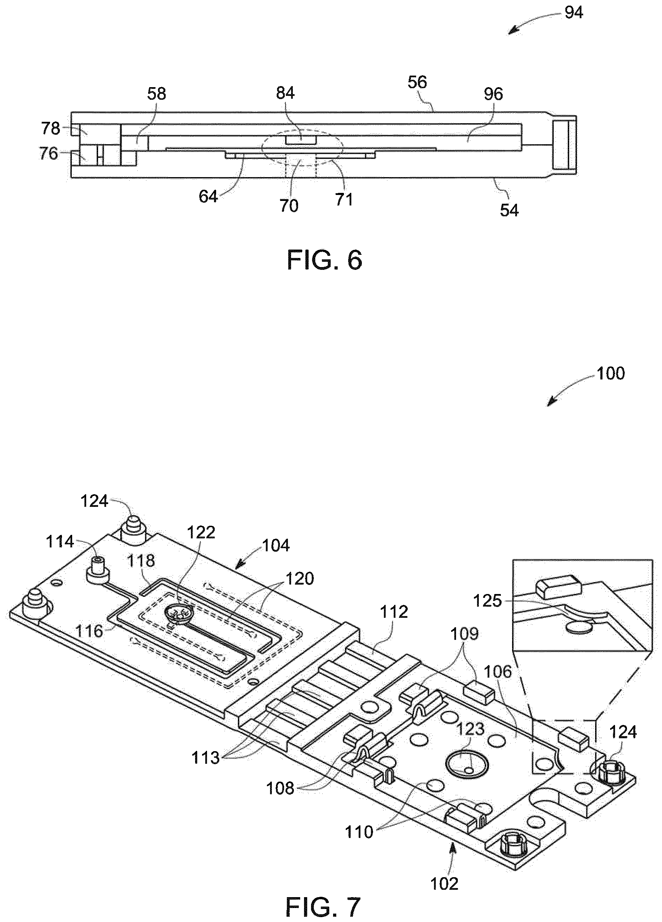

As discussed below with regard to FIG. 7, in some embodiments, the secondary member may be disposed in the substrate frame. However, in other embodiments, the substrate frame 54 may form part of an external device, or may be provided separately. The compression member 84 may be configured to provide an isolation zone on the sample substrate 64, where the isolation zone is generally represented by reference numeral 71. The substrate frame 54 may include a clearance hole 72 for an inlet port 82, disposed in the substrate cover 56 or the laminate layer 57, when the sample storage and extraction device is in a folded configuration. The inlet port 82 is in turn connected to the fluidic channel 73 disposed in the substrate cover 56 or the laminate layer 57 (see FIG. 5). In operation, having the inlet port 82 and the exit port clearance 70 on the same side of the sample storage and extraction device 50 allows an easier connection to the external fluidic device. During elution, the substrate 64 is compressed between the compression member 84 and the secondary member.

As discussed in detail with respect to FIG. 6, the compression member 84 may be disposed in the substrate cover 56 and the secondary member may be disposed in the substrate frame 54 or the external device. In instances where the secondary member of the compression assembly is part of the external fluidic device, the secondary member of the compression assembly may be configured to access the sample substrate 64 through the opening of the exit port clearance 70. In instances where the secondary member of the compression assembly is desposed in the substrate frame 54, the secondary member of the compression assembly may be in the same position as the exit port clearance 70 shown in Figure 4. In one embodiment, the compression member 84, the secondary member, or both may include a compression ring. The members of the compression assembly may be configured to press against opposite sides of the sample substrate 64 and allow fluid to flow through a defined area 71 of the sample substrate 64.

Further, the substrate frame 54 may also include standoffs 74 configured to maintain air gap between the substrate frame 54 and substrate cover 56, when the sample storage and extraction device 50 is folded, for example, to store the sample for analysis. The substrate frame 54 and substrate cover 56 may further include standoffs 76 and 78, respectively, to maintain air gap between the substrate frame 54 and substrate cover 56 when the device 50 is folded. Additionally, the substrate cover 56 may include snap features 80 that in conjunction with the standoffs 74 maintain the closed state of the folded sample storage and extraction device 50. The snap features 80 may correspond to the standoffs 74 present on the substrate frame 54.

In the illustrated embodiment, reference numeral 59 may be used to represent the combination structure having the substrate cover 56 and the laminate layer 57. In one embodiment, the combination structure 59 may include a flexure 86. The flexures 86 may be configured to apply uniform force on the sample substrate 64 to form an isolated region (not shown) in a portion of the sample substrate 64. In an example embodiment, the flexures 85 and 87 of the flexure 86 may form a double-flexure design. In one embodiment, the compression member 84 may be disposed on a double-backed flexure 86 to provide a low compliance structure that only contacts the substrate 64 when a load is applied.

In some embodiments, a load may be applied to the compression member 84 in the folded state of the device 50 to form an isolation zone in the substrate 64. The compression member 84 is configured to isolate a given area of the sample substrate 64 such that fluid flow is directed through the area of interest. The effectiveness of the isolation (i.e., minimizing lateral wicking through the sample substrate 64) may depend on the sealing component geometry and the applied force. Additionally, the sample substrate material, applied sample, elution solution and solutes, and fluid residence time (i.e., flow rate) may also affect the effectiveness of the sealing. In certain embodiments, the flexure 86 is configured to provide a structure that is configured to withstand applied loads needed for creating isolation zone 71 on the sample substrate. In some embodiments, features, such as a double-backed flexure, structural fluid path, may be added to enhance symmetry of the applied pressure.

FIG. 5 illustrates details of the structure disposed below the laminate layer 57 (see FIG. 4) of the substrate cover 56 of the sample storage and extraction device 50. In the illustrated embodiment, the double-flexured design of the flexure 86 facilitates the compression member 84 to be disposed parallel to the corresponding portion of the substrate 64 to form the isolation zone in the folded configuration of the sample storage and extraction device 50. In the illustrated embodiment, the substrate cover 56 may include a fluidic channel 73 to provide reagent fluid to the isolated region. Additionally, one or more balancing structures 88 may be employed in the sample storage and extraction device 50 to facilitate enhanced balanced loading in radial and circumferential directions of the compression member 84. Moreover, although not illustrated additional balancing structures may be located under the compression member 84 to enhance the uniformity of the pressure distribution of a sealing surface between the compression member 84, the substrate 64, and the external device (not shown). The balancing structures 88 may be any mechanical design that facilitates even distribution of the pressure in the sealing surface defined by the compression member 84. In a non-limiting example, the balancing structures 88 may include artificial fluid paths.

FIG. 6 illustrates a side view 94 of the sample storage and extraction device 50 of FIGS. 4 and 5 seen in a direction generally represented by arrow 90 (see FIG. 4). As illustrated, an air gap 96 is maintained between the sample substrate 64 and the substrate cover 56. Further, the compression member 84 is aligned with the exit port 70.

FIG. 7 illustrates an alternative embodiment of the sample storage and extraction device 50 of FIGS. 4-5. The sample storage and extraction device 100 of FIG. 7 includes a substrate frame 102 and a substrate cover 104. The substrate cover 104 may or may not include a laminate layer. The substrate frame 102 may include a region 106 for receiving a sample substrate (not shown). The substrate frame 102 may include a secondary member 123, and the substrate cover 104 may include a compression member 122. The compression member 122 and the secondary member 123 may together form a compression assembly. In the illustrated embodiment, the compression member 122 is in the form of a compression ring. The secondary member 123 is positioned such that when the device 100 is closed after sample collection, the secondary member 123 aligns with the compression member 122 disposed on the substrate cover 104.

The substrate frame 102 may include a plurality of standoff structures 110 configured to provide suitable air gap between the substrate frame 102 and substrate cover 104. Further, the substrate frame 102 may include a plurality of clips 108. The clips are configured to hold the sample substrate in place in the substrate frame 102 without the need for any pressure sensitive adhesive. It may be noted that avoiding the use of the pressure sensitive adhesive reduces the chances of having any adverse effect on the device 100 that may otherwise be present due to degradation of the pressure sensitive adhesive and allows for longer shelf-life of the device 100. The clips 108 are configured to receive the sample substrate and coupled the sample substrate with the substrate frame 102. A boss 109 may be provided on a side of each clip 108, such that the boss 109 is disposed outside the sample region. Further, the boss 109 is configured to prevent accidental pressing of the clips 108 when the device 100 is closed. As will be understood, accidental pressing of one or more clips 108 may dislodge the sample substrate from its position.

The sample storage and extraction device 100 is configured to be compatible with various designs of sample collection devices (not shown). Further, the sample storage and extraction device 100 includes a living hinge 112. The living hinge 112 may include a plurality of portions 113 that may together provide reversible folding and unfolding of the substrate frame 102 and the substrate cover 104. In one embodiment, a size of the living hinge 112 may be adjusted for optimal flexibility configuration.

Moreover, the sample storage and extraction device 100 is configured to easily receive and couple the sample substrate to the sample substrate region 106. By way of example, the sample storage and extraction device 100 may include fasteners, such as, but not limited to, clips 107, to hold a sample substrate (not shown). The device 100 may further include an inlet port 114, a fluidic channel 116, a balancing structure 118, flexure 120, and provisions 124 on the substrate frame 102 and the substrate cover 104 for locking the substrate frame 102 and substrate 104 together. A portion of the sample storage and extraction device, such as the substrate cover 104, may include an inlet port for inlet connection of the sample substrate to an external the fluidic source. A slot 125 may be present in the region 106 to receive a portion of the sample substrate. In one example, a portion of the sample substrate may be first disposed in the slot 125, and the remaining portion of the sample substrate may then be laid on the region 106.

In certain embodiments, a method for processing and analyzing samples disposed on a portion of a sample substrate disposed on a substrate frame may include creating a compression seal to form an isolation zone at least in a portion of the sample substrate having the sample, applying a fluid to the portion of the sample isolated in the isolation zone by flowing the fluid through the isolation zone, collecting at least a portion of the fluid after it is flowed through the isolation zone, clearing the fluid from the isolation zone by flowing gas through the isolation zone, releasing the compression seal, and analyzing one or both of the collected fluid and the portion of the sample in the isolation zone.

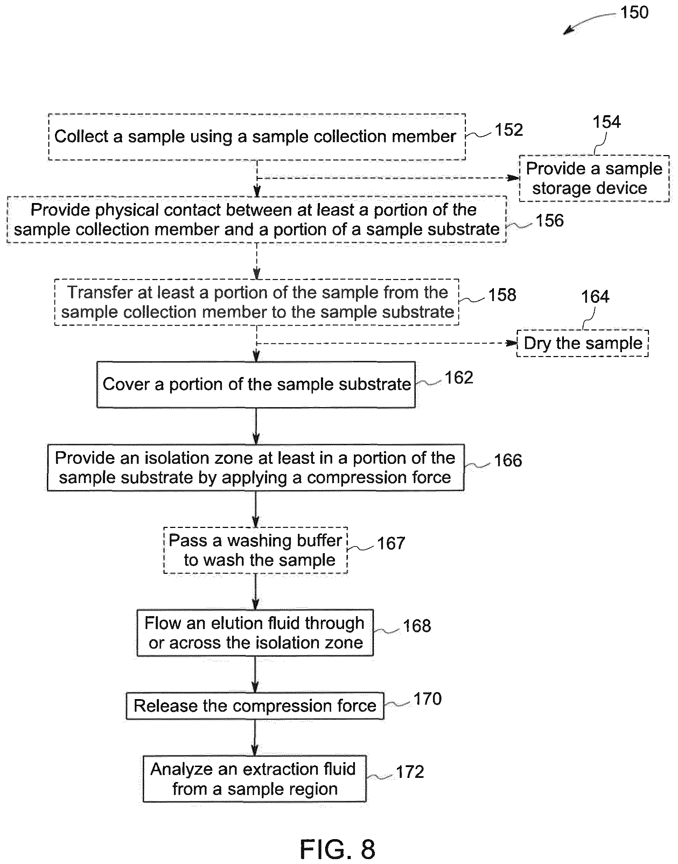

FIG. 8 illustrates a flow chart 150 of an example method for collecting a sample, transferring the sample to a sample substrate, storing the sample for analysis, and analyzing the sample. At block 152, the method may commence by collecting a sample using a collection member. In one embodiment, the collection member forms part of a sample collection device. In one embodiment, the sample collection member may be an integral part of the sample collection device. In another embodiment, the sample collection member may be removably coupled to the sample collection device. In this embodiment, the sample collection member may or may not be coupled to the sample collection device during collection of the sample. In some embodiments, the sample collection device and a sample storage and extraction device may form an integral monolithic structure. Whereas, in another embodiment, the sample collection device and the sample storage and extraction device may be removably coupled to one another.

Optionally, at block 154, in embodiments where the sample collection and the sample storage and extraction device do not form an integral structure, a sample storage and extraction device may be provided. The step of providing the sample storage and extraction device may include disposing a sample substrate in the sample substrate holder of the sample storage and extraction device. Moreover, in embodiments where the sample collection and the sample storage and extraction device do not form an integral structure, the sample collection device may be coupled to the sample storage and extraction device after collecting the sample. The sample storage and extraction device may include a sample storage and extraction device. The step of coupling the sample storage and extraction device to the sample collection device may include operatively coupling the sample storage and extraction device to the sample collection device.

At block 156, a physical contact may be provided between at least a portion of the sample collection member and the sample substrate. Referring back to FIGS. 2-3, in some embodiments, when the sample storage and extraction device 10 is coupled to the sample collection device, the substrate cover 13 may be configured to slide back, thereby exposing the sample substrate 14 to the sample collection member.

At block 158, at least a portion of the sample may be transferred from the sample collection member to the sample substrate. The transfer of the sample from the sample collection member to the sample substrate may be facilitated by applying a determined amount of pressure on the sample collection member and rotational movements of the sample collection member. In one example, the pressure applied to the sample collection member may enable the sample collection member to bend at a determined angle with respect to the sample substrate, thereby increasing the contact surface between the sample collection member and the sample substrate. The positioning of the sample on the sample substrate may be such that the sample area or extraction area on the sample substrate is aligned with the sealing features of the compression assembly and the connected instrument, such as an external device.

Optionally, the sample storage and extraction device may be decoupled from the sample collection device. At block 162, at least a portion of the sample substrate having the substrate may be covered. In one example, the substrate may be covered with the substrate cover. In particular, the sample substrate may be closed immediately before or after decoupling the sample substrate frame from the sample collection device. In an example embodiment, upon decoupling the substrate cover 13 (see FIG. 3) may be configured to slide on the holder base 12 (see FIG. 3) to cover at least the portion of the sample substrate having the sample.

Optionally, at block 164, the sample may be allowed to dry for a determined period of time. Further, the sample storage device may be dispatched to a desirable location or stored in the lab for analysis of the sample.

Steps 166-172 are example steps for processing and analyzing the sample disposed on the sample substrate of the sample storage and extraction device. At block 166, the processing of at least a portion of the sample disposed on the sample substrate may include providing an isolation zone at least in a portion of the sample substrate. In some embodiments, the isolation zone is created using a compression seal. In an example embodiment, the step of creating the compression seal may include forming one or more isolation zones on the sample substrate. The step of creating the compression seal may also include compressing a compressible membrane of filter along with the sample substrate.

The isolation zone facilitates isolating the portion of the sample substrate having the sample from the rest of the sample substrate. In a non-limiting example, the sealing features (84) may compress the sample substrate, thereby forming a seal which prevents liquids, such as an extraction buffer, that are introduced to the isolation zone, via a fluid inlet, from wicking outward from the initial point at which the buffer is applied to the sample substrate.

At block 168, an elution fluid may flow through or flow across the isolation zone of the sample substrate to extract the sample from the sample substrate at the time of analysis. One or more fluids may be applied to the sample simultaneously or serially. A determined amount of external force may be applied to seal the fluid channel against the sample substrate for flow through/across elution of the region of interest. To minimize wicking of the elution fluid, the substrate frame may create an isolation zone on the sample substrate. The sample in the isolation zone may be exposed to a fluid. The fluid may be flown through the isolation zone using microfluidics. After flowing the fluid or while flowing the fluid through at least a portion of the isolation zone, at least a portion of the fluid is collected after it is flowed through the isolation zone. The outgoing fluid from the isolation zone may be cleared by flowing a gas through the isolation zone.

Optionally, as illustrated by block 167, a washing buffer may be passed to wash the sample. In one embodiment, the washing buffer may be passed through the sample to remove unwanted contaminants. In one embodiment the washing buffer may include a solution containing about 50% to about 90% by volume of alcohol (e.g., EtOH)

In some embodiments, an extraction fluid or buffer is applied to the sample substrate through an inlet tube. At least a portion of the buffer may flow through the paper, without wicking outside the isolation zone. The buffer may flow out via the outlet tube. In the illustrated embodiment, the outlet tube may be concentrically located in the isolation zone. The outlet tube may have an outlet in a receptacle such as a well plate or a vial. The outlet tube may feed directly into analysis instrumentation. In one example, air may be introduced into and forced through, the device or system to remove any remaining liquid or foreign materials within the fluid path, while the compression force is being applied. Air may also be introduced to remove excess fluid from the sample area to dry the location and prevent wicking of fluids after the compression force is released.

At block 172, an extraction fluid flowed out through the sample region may be analyzed. The step of analyzing may include identifying one or more components of the sample. Further, the step of analyzing may include quantifying an amount of one or more substances in the collected fluid. In some embodiments, one or both of the collected fluid and the portion of the sample in the isolation zone may be analyzed. The analyzing step may include quantifying an amount of one or more substances in the collected fluid. In methods in which the sample comprises blood or other various types of biological materials, the analyzing step may comprise identifying one or more components of the sample.

The methods and systems of the disclosure may analyze the samples and materials extracted from the samples for many different purposes using a variety of analyzing systems such as, but not limited to, immunoassays (e.g. to identify the presence or absence of a component), liquid chromatography with UV detection (e.g. to characterize and quantify components), qPCR, RT-PCR, DNA microarrays, isothermal nucleic acid amplification and liquid chromatography with mass spectrometry (e.g. to identify and/or quantify components).

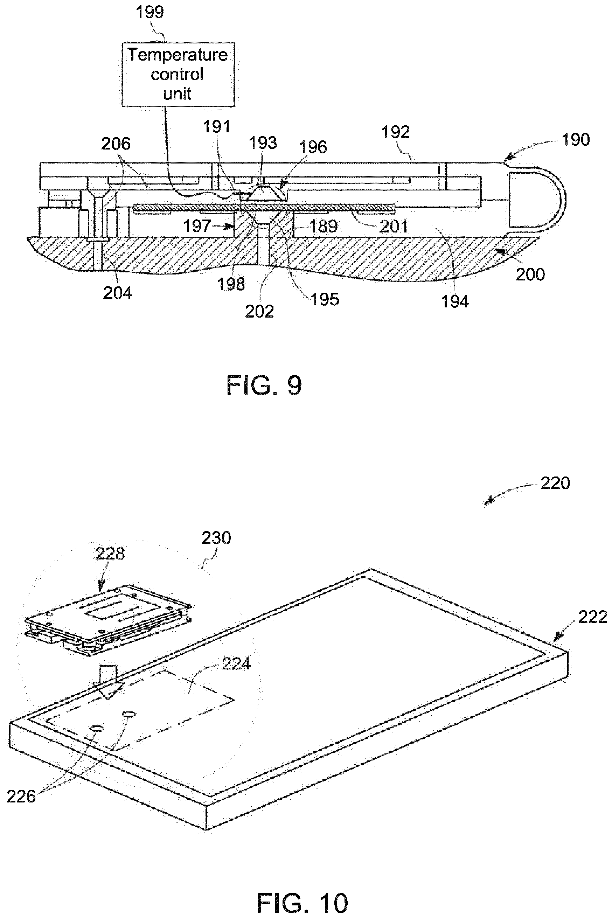

Turning now to FIG. 9, a sample storage and extraction device 190 is illustrated in a folded state and shown in cross-section through the long-axis centerline of the folded assembly. The sample storage and extraction device 190 is coupled to an external device 200. The sample storage and extraction device 190 includes a substrate cover 192 disposed on a substrate frame 194. Further, a compression member 193 is disposed in the substrate cover 192, and a secondary member 195 is disposed in the substrate frame 194. The compression member 193 and the secondary member 195 together form a compression assembly 196. Further, the secondary member 195 may be operatively coupled to a boss 197. In one embodiment, the boss may be part of the substrate frame 194. In this embodiment, the boss 197 may be configured to be decoupled from the external device at the location 189 represented by a dashed line. In another embodiment, the boss 197 may be a part of the external device 200.

The compression assembly 197 is configured to provide an isolation zone on the sample substrate. The isolation zone is created by pressing the members 193 and 195 of the compression assembly 197 on opposite sides of the sample substrate 201. When providing isolation zone on the sample substrate 201, the compression member 193 may be aligned with the secondary member 195Assuming standard manufacturing tolerances, the design of dual (inlet/outlet) port connection with low hold-up volume is challenging. However, by creating a compression assembly 197 having one part (compression member 193) in the sample storage and extraction device 190, and another part (secondary member 195) in the external device 200, where the secondary member 195 includes the boss 197 of the external device 200, the hold-up volume (of eluent sample) in the outlet side of the substrate frame 194 may be effectively reduced to zero. In the illustrated embodiment, a face-seal (not shown) may be used on the inlet-side and accounting for assembly and part tolerances the hold-up volume may be less than or equal to about 2.5µl. In operation, a fluid connection may be established between the sample storage and extraction device 190 and the external device 200. The inlet sealing pressure is applied by an external load, similar to the compression seal. The external device 200 is configured such that an elution port 202 is aligned with the hole 198 in the external device 200 and aligned with the compression member port 195.. Additionally, the external device 200 may include an elution inlet or buffer inlet 204 that is aligned with a fluid path 206 present in the sample storage and extraction device 190.

The arrangement of FIG. 9, further includes a temperature sensor 191 operatively coupled to a temperature control unit 199. In one example, the temperature sensor 191 may include a thermocouple. The thermocouple may be embedded in a small chamber disposed in proximity to the compression assembly 196 to measure fluid temperature.

Referring to FIG. 10, a system 220 includes a sample storage and extraction device 228 in a folded state. The sample storage and extraction device 228 is configured to be coupled to a fluidic device assembly 222. The fluidic device assembly 222 may include an external device holder 224 configured to receive the sample storage and extraction device 228. The device holder 224 includes alignment structures 226. The alignment structures 226 are configured to interface with the sample storage and extraction device 228 and hold the sample storage and extraction device 228 in a relative location such that the second part of the compression seal and the fluidic connection to the inlet are aligned with the inlet and outlet connections of the sample storage and extraction device 228. In one embodiment, the alignment structures 226 may include pins, or other suitable fasteners. Alternatively, the sample storage and extraction device 228 may be coupled to the fluidic device assembly 222 or the other external devices using fasteners other than the alignment structures 226. Further, it should be noted that FIG. 10 illustrates a specific example, where the sample storage and extraction device 228 is interfaced with the fluidic device assembly 222, however, it should be noted that the sample storage and extraction device 228 may be interfaced with other external devices, such as, other external fluidic devices or analysis instruments.

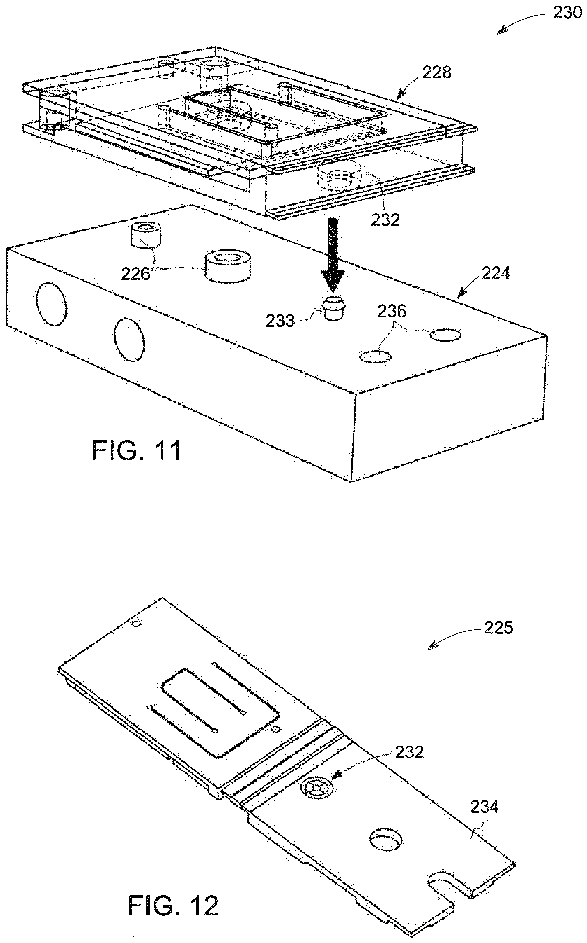

FIG. 11 illustrates a detailed view of a portion 230 of the system 220 of FIG. 10. FIG. 12 illustrates a perspective view 225 of an example sample storage and extraction device 228 configured to be operatively coupled to the external device 224. In the illustrated embodiments of FIGS. 11-12, the external device 224 includes bosses 226, an alignment structure 233, and through holes 236. The bosses 226 may be configured to act as alignment features during coupling of the sample storage and extraction device 228 and the external device 224. Further, the boss 226 disposed closer to the sample substrate may form a part of the compression assembly. Also, the boss 226 disposed farther away from the sample substrate may form a part of the inlet assembly. The alignment structure 233 is configured to be coupled to corresponding structure(s) 232 disposed in the sample storage and extraction device 228. In the illustrated embodiment, the corresponding structure 232 is disposed in a substrate frame 234 of the sample storage and extraction device 228. Further, the external device 224 may include the through holes 236 for receiving alignment pins of the sample storage and extraction device 228. The alignment pins may interface with the external device 224 and hold the external device 224 in a desirable location such that any apparatus applying force to the area above the compression assembly is properly aligned..

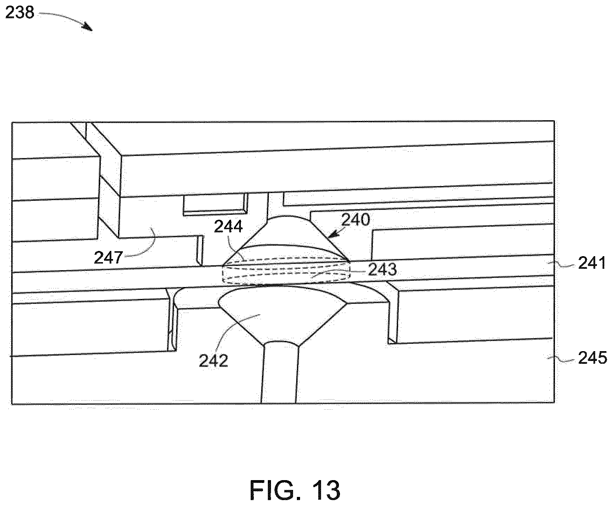

FIG. 13 illustrates a portion of a sample storage and extraction device 238 employing a compression assembly 240 configured to provide a compression seal to form an isolation zone 243 in at least a portion of a sample substrate 241 such that the fluid flow is directed through the isolation zone 243. The effectiveness of the isolation (i.e., minimizing lateral wicking through the substrate) is dependent on the sealing component geometry and the applied force. Additionally, the substrate material, applied sample, elution solution and solutes, and fluid residence time (i.e., flow rate) also may affect the sealing effectiveness. The illustrated embodiment depicts the compression assembly 240 having a compression member 244 and a secondary member 242. The compression member is disposed in a substrate cover 207, and the secondary member 242 is disposed in an external device 245.

In operation, the compression and secondary members 244 and 242 may be configured to effectively press against a portion of the sample substrate 241 disposed between the compression and secondary members 244 and 242 of the compression assembly 240. Upon application of the pressure to the sample storage and extraction assembly 228 the compression assembly 240 may provide a compression seal to form the isolation zone 243. Further, the isolation zone 243 formed by the compression assembly 240 allows analysis of a sample disposed in the isolation zone 243 without the need to cut and capture pieces of the sample substrate 241 corresponding to the isolation zone 243. In one embodiment, the compression and secondary members 244 and 242may be separate individual components that may be directly attached to a sample storage and extraction device when required. In another embodiment, one or both the compression and secondary members 244 and 242 may form integral parts of the sample storage and extraction device. In one embodiment, the compression member 244 may be a disposable component. It should be noted that the sealing design directly affects the force required to ensure effective sealing, which is a function of the peak pressure in the sealing area and the distance the fluid needs to travel through the pressure field.

Moreover, it should be noted that it is desirable to reduce the value of force required to provide the compression seal because the disposable sample storage and extraction device may be manufactured using materials, such as, but not limited to, polypropylene, nylon, acrylonitrile butadiene styrene (ABS), or combinations thereof, that are economically viable.

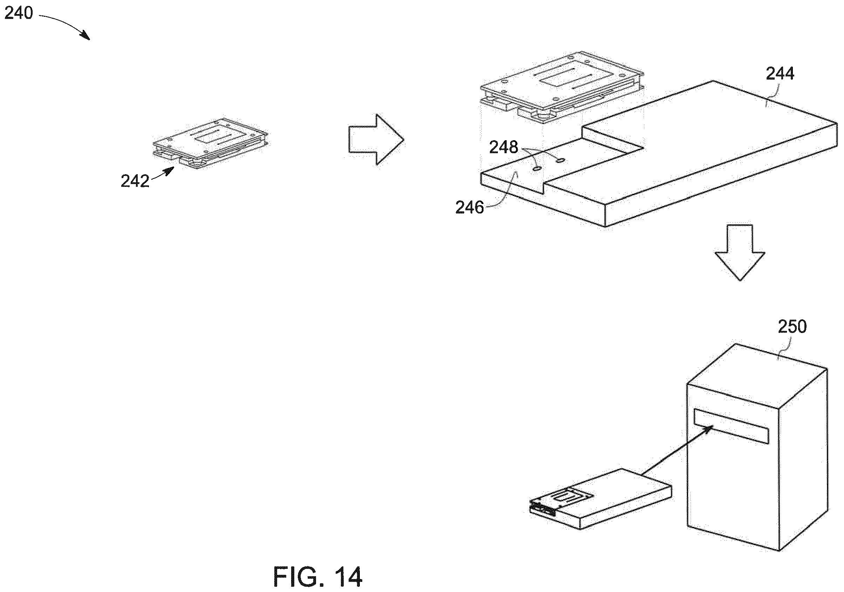

Referring now to FIG. 14, an example workflow 240 for sample analysis using a sample storage and extraction device 242 of the present disclosure is illustrated. In the illustrated embodiment, the sample storage and extraction device 242 having the covered sample substrate with the transferred sample is coupled to a fluidic device 244. The sample storage and extraction device 242 may be coupled to the fluidic device 244 by disposing the sample storage and extraction device 242 in a recess 246 having provisions 248 to receive the sample storage and extraction device 242. The fluidic device 244 having the sample substrate frame 242 may then be coupled to the analysis instrument 250 to analyze the sample. In one example, the provisions 248 may be an intuitive snap-in interface between the sample substrate frame 242 and the fluidic device 244. The design of the sample storage and extraction device 242 enables analyzing the sample disposed in the sample storage and extraction device 242 without bringing the sample in undesirable physical contact with the user, or surfaces or devices, etc.

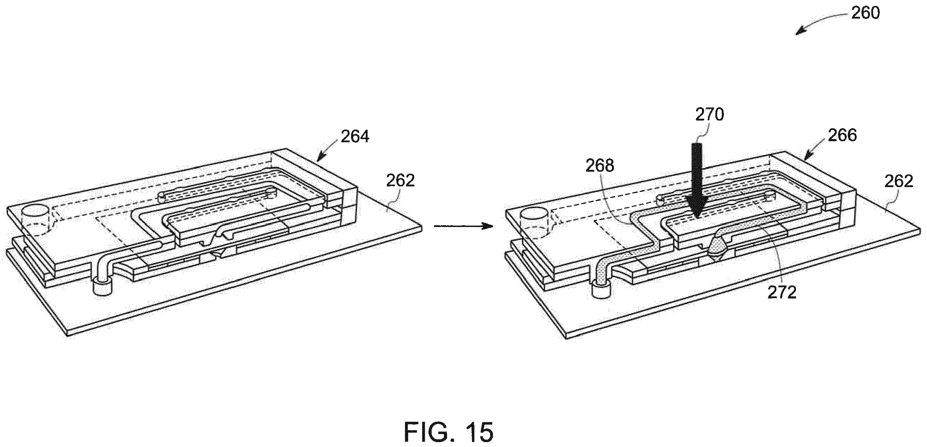

FIG. 15 illustrates an arrangement 260 before and after a fluidic communication between an external device, such as a fluidic device 262 and sample storage and extraction device 264 is established. Reference numeral 266 generally represent the sample storage and extraction device 264 in operation, where a fluidic communication between the fluidic device 262 and the sample storage and extraction device 264 may be established by applying a force on the compression seal in a direction generally represented by arrow 270. The force moves the two parts of the compression seal closer together so that they compress the substrate between them. The fluidic communication between the fluidic device 262 and the sample storage and extraction device 266 is established after forming the isolation zone. A reagent fluid 272 may be delivered to the isolation zone using a fluidic channel 268 of the sample storage and extraction device 264. The reagent fluid 272 may be primarily confined to the isolation zone, without any substantive wicking of the portions of the sample substrate disposed outside the isolation zone. After flowing the reagent fluid 272 in the isolation zone, the compression seal may be released, and one or both of the collected reagent fluid and the portion of the sample in the isolation zone may be analyzed.

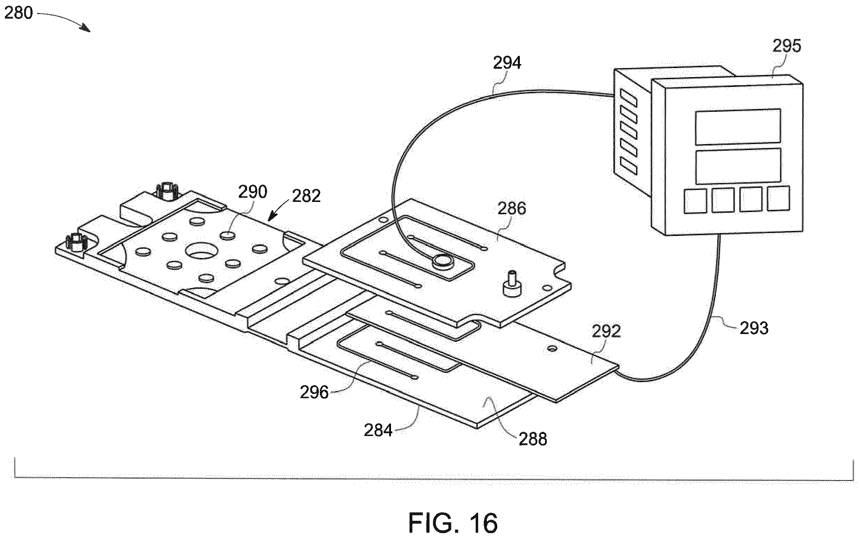

FIG. 16 illustrates an arrangement 280 where a sample storage and extraction device 282 is operatively coupled to a heating element 292. The heating element 292, in turn, may be connected to a temperature control unit 295 using electrical connections 293. In particular, the temperature control unit 295 may be operatively coupled to a fluidic channel (not shown). For example, the heating element 292 may be operatively coupled to an inlet port (not shown) of the fluidic channel. The heating element 292 may be configured to heat the elution solution to a desirable temperature in a controlled manner before the elution solution interacts with the sample. Heating the elution solution before elution may positively affect the concentration of analytes in the elute. The heating element 292 may be designed to be disposed in a substrate cover 284 of the sample storage and extraction device 282. In some embodiments, the temperature control unit 295 may be configured to adjust a temperature of the heating element 292. In addition to being coupled to the heating element 292, the temperature control unit 295 may be further coupled to a temperature sensor (not shown) disposed in the sample extraction and storage device 282. In the illustrated embodiment, the temperature control unit 295 may be coupled to the temperature sensor using electrical connection 294. In one example, the temperature sensor may include a thermocouple. The thermocouple may be embedded in a small chamber disposed in proximity to the compression assembly to measure fluid temperature.

In one example, the heating element 292 may be disposed under a lamination 296 of the substrate cover 284. In a non-limiting example, the heating element 292 is a thin-film multi-layer flexible heater made of polyimide outer layers and nichrome traces and is laminated between the body 288 and the lamination layer 296 of the substrate cover. In this example, the heating element 292 may be disposed between the body 288 and the lamination layer 296 along with the fluidic channel (not shown). In one embodiment, the heating element 292 may be directly coupled to the elution solution to enhance the heating efficiency. In one example, a temperature sensor is used in conjunction with the temperature control unit 194 to detect the temperature of the elution solution. By way of example, the temperature sensor may be embedded in the heating element 292 to measure the temperature of the elution solution. In one embodiment, a miniature temperature sensor, e.g., a thermocouple may be disposed at a determined distance above a sample substrate 290 to accurately measure the elution solution temperature in the elution process. Although not illustrated, the heating element 292 may include provisions (e.g., slits) that match in alignment to flexures 296. In a particular example, the electrical connections 294 to the temperature control unit 295 and sensor leads may be connected to the corresponding leadings in the sample storage and extraction device using exposed contacts (storage device side) and spring loaded pins (on the side of the external device).

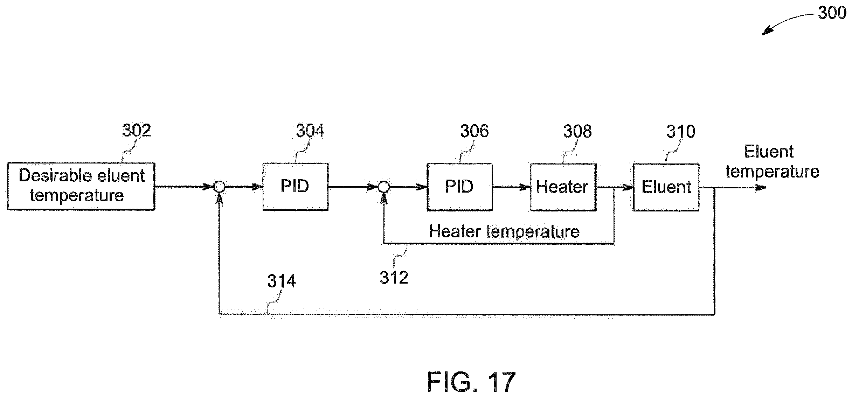

FIG. 17 illustrates an example flow diagram 300 for achieving a desirable elution solution temperature. A desirable temperature of the elution solution is provided as an input 302 to a proportional-integral-derivative controller (PID) controller 304. In one example, a cascading PID controller 304 may be used to control the temperature of the elution solution. The PID controller 304 may be operatively coupled to another PID controller 306. The PID controller 306 is in turn coupled to a heater 308 to provide elution solution 310 at a desirable temperature to the sample. The inner loop 312 modulates the temperature of the heater 308 to prevent over heating near the heater surface which may result in micro-bubbling that inhibits the flow of the elution solution to the isolation zone. The outer loop 314 controls the temperature of the elution solution.

In some embodiments, for record keeping and traceability the present device may also comprise an identification label (such as conventional bar coding). In one example, the identification label may be disposed on the sample collection device and the sample storage and extraction device.

Advantageously, the sample storage and extraction device of the present disclosure is user friendly and easy-to-use in point of care systems that may require one or more of sample collection, sample transfer, sample storage, elution through the sample substrate, and device integration. The single-use and disposable nature of the sample storage and extraction device prevents or minimizes transfer of infection between users and transfer of analytes between samples.

While only certain elements of the invention have been illustrated and described herein, many modifications and changes will occur to those skilled in the art.

## Claims

1. A sample storage and extraction device (10), comprising:
a substrate frame (54) having a substrate region configured to receive a sample substrate (62) having a sample region;
a substrate cover (56) operatively coupled to the substrate frame;
a compression assembly (34) configured to provide an isolation zone; and
a fluidic channel (26) configured to flow an elution fluid to the isolation zone, the device being **characterised in that** the isolation zone is provided in a portion of the sample substate and the compression assembly includes a compression member disposed on a flexure said flexure being configured to be disposed around the sample region of the sample substrate, wherein the flexure is further configured to distribute an applied force to form the isolation zone.

2. The sample storage and extraction device of claim 1, wherein the compression assembly further comprises a compression member, for example a compression ring, and a secondary member, wherein the compression member is disposed in the substrate frame, and wherein the secondary member is disposed in the substrate cover.

3. The sample storage and extraction device of claim 1 or 2, wherein: the substrate frame, substrate cover, or both comprise one or more gap standoffs; and/or wherein the substrate cover comprises an inlet port; and/or wherein the substrate frame and the substrate cover comprise an inlet port and an outlet port, respectively; and/or wherein the substrate frame comprises a hole corresponding to a position of a compression member in the substrate cover.

4. The sample storage and extraction device of claim 1, wherein the flexure comprises a double-flexure design.

5. The sample storage and extraction device as claimed in any one of claims 1 to 4, further comprising a heating element optionally in the form of a thin-film multi-layer flexible heater.

6. A method, comprising:
providing a sample storage and extraction device as claimed in any one of claims 1 to 5 including a substrate frame and a substrate cover;
disposing a sample substrate in a portion of the substrate frame;
providing an isolation zone in a portion of the sample substrate by applying a compression force on the sample substrate in a determined direction;
flowing an elution fluid through or across the isolation zone;
releasing the compression force; and
analyzing a portion of the elution fluid flowed out of the isolation zone.

7. The method of claim 6, wherein the step of providing the isolation zone comprises isolating a portion of the sample substrate having a sample from other portions of the sample substrate.

8. The method of claim 6 or 7, wherein the step of providing the isolation zone comprises compressing one or more sealing features of a compression assembly, for example a compression ring assembly, against the sample substrate.

9. The method of claims 6,7 or 8, wherein providing the isolation zone comprises applying a determined amount of pressure on the substrate cover to form a compression sealing.

10. The method of claims 6,7,8 or 9, wherein flowing the elution fluid through or across the isolation zone comprises flowing the elution fluid through or across the isolation zone using a fluidic channel disposed in the substrate cover.

11. The method of claim 10, further comprising heating the elution fluid before flowing the elution fluid through or across the isolation zone.

12. The method of any one of claims 6 to 11, further comprising operatively coupling the sample storage and extraction device to an external device.

13. A system, comprising:
a sample storage and extraction device as claimed in any one of the claims 1 to 5,
wherein the system further comprises a heating element operatively coupled to a control unit.

## Patentansprüche

1. Probenlagerungs- und Extraktionsvorrichtung (10), umfassend:
einen Substratrahmen (54), der eine Substratregion aufweist, die ausgelegt ist, um ein Probensubstrat (62) aufzunehmen, das eine Probenregion aufweist;
eine Substratabdeckung (56), die betriebsmäßig mit dem Substratrahmen gekoppelt ist;
eine Kompressionsanordnung (34), die ausgelegt ist, um einen Isolationsbereich bereitzustellen; und
einen fluidischen Kanal (26), der ausgelegt ist, um ein Elutionsfluid zum Isolationsbereich zu strömen, wobei die Vorrichtung **dadurch gekennzeichnet ist, dass** der Isolationsbereich in einem Abschnitt des Probensubstrats bereitgestellt ist und die Kompressionsanordnung ein Kompressionselement enthält, das an einer Krümmung angeordnet ist, wobei die Krümmung ausgelegt ist, um um die Probenregion des Probensubstrats herum angeordnet zu sein, wobei die Krümmung weiter ausgelegt ist, um eine angelegte Kraft zu verteilen, um den Isolationsbereich zu bilden.

2. Probenlagerungs- und Extraktionsvorrichtung nach Anspruch 1, wobei die Kompressionsanordnung weiter ein Kompressionselement, zum Beispiel einen Kompressionsring, und ein sekundäres Element umfasst, wobei das Kompressionselement im Substratrahmen angeordnet ist, und wobei das sekundäre Element in der Substratabdeckung angeordnet ist.

3. Probenlagerungs- und Extraktionsvorrichtung nach Anspruch 1 oder 2, wobei: der Substratrahmen, die Substratabdeckung oder beide einen oder mehrere Spaltabstände umfassen; und/oder wobei die Substratabdeckung einen Einlassanschluss umfasst; und/oder wobei der Substratrahmen und die Substratabdeckung jeweils einen Einlassanschluss und einen Auslassanschluss umfassen; und/oder wobei der Substratrahmen ein Loch umfasst, das einer Position eines Kompressionselements in der Substratabdeckung entspricht.

4. Probenlagerungs- und Extraktionsvorrichtung nach Anspruch 1, wobei die Krümmung ein Doppel-Krümmungsdesign umfasst.

5. Probenlagerungs- und Extraktionsvorrichtung nach einem der Ansprüche 1 bis 4, weiter umfassend ein Heizelement, das optional in der Form eines viellagigen dünnschichtigen flexiblen Heizers ist.

6. Verfahren, umfassend:
Bereitstellen einer Probenlagerungs- und Extraktionsvorrichtung nach einem der Ansprüche 1 bis 5, enthaltend einen Substratrahmen und eine Substratabdeckung;
Ablegen eines Probensubstrats in einem Abschnitt des Substratrahmens;
Bereitstellen eines Isolationsbereichs in einem Abschnitt des Probensubstrats durch Anlegen einer Kompressionskraft an das Probensubstrat in eine vorbestimmte Richtung;
Strömen eines Elutionsfluids durch oder über den Isolationsbereich;
Entlasten der Kompressionskraft; und
Analysieren eines Anteils des durch den Isolationsbereich geströmten Elutionsfluids.

7. Verfahren nach Anspruch 6, wobei der Schritt des Bereitstellens des Isolationsbereichs Isolieren eines Abschnitts des Probensubstrats umfasst, der eine Probe von anderen Abschnitten des Probensubstrats aufweist.

8. Verfahren nach Anspruch 6 oder 7, wobei der Schritt des Bereitstellens des Isolationsbereichs Komprimieren von einer oder mehreren Dichtungsmerkmalen einer Kompressionsanordnung, zum Beispiel einer Kompressionsringanordnung, gegen das Probensubstrat umfasst.

9. Verfahren nach Ansprüchen 6,7 oder 8, wobei Bereitstellen des Isolationsbereichs Anwenden einer bestimmten Menge von Druck auf die Substratabdeckung umfasst, um eine Kompressionsdichtung zu bilden.

10. Verfahren nach Ansprüchen 6,7,8 oder 9, wobei Strömen des Elutionsfluids durch oder über den Isolationsbereich Strömen des Elutionsfluids durch oder über den Isolationsbereich unter Verwenden eines fluidischen Kanals umfasst, der in der Substratabdeckung angeordnet ist.

11. Verfahren nach Anspruch 10, weiter umfassend Erhitzen des Elutionsfluids vor dem Strömen des Elutionsfluid durch oder über den Isolationsbereich.

12. Verfahren nach einem der Ansprüche 6 bis 11, weiter umfassend betriebsmäßiges Koppeln der Probenlagerungs- und Extraktionsvorrichtung mit einer externen Vorrichtung.

13. System, umfassend:
eine Probenlagerungs- und Extraktionsvorrichtung nach einem der Ansprüche 1 bis 5,
wobei das System weiter ein Heizelement umfasst, das betriebsmäßig mit einer Steuereinheit gekoppelt ist.

## Revendications

1. Dispositif de stockage et d'extraction d'échantillon (10) comprenant :
un cadre de substrat (54) présentant une région de substrat configurée pour recevoir un substrat d'échantillon (62) présentant une région d'échantillon ;
un couvercle de substrat (56) couplé en fonctionnement au cadre de substrat ;
un ensemble de compression (34) configuré pour fournir une zone d'isolation ; et
un canal fluidique (26) configuré pour faire écouler un fluide d'élution à la zone d'isolation, le dispositif étant **caractérisé en ce que** la zone d'isolation est prévue dans une portion du substrat d'échantillon et l'ensemble de compression inclut un élément de compression disposé sur une flexion, ladite flexion étant configurée pour être disposée autour de la région d'échantillon du substrat d'échantillon, dans lequel la flexion est en outre configurée pour distribuer une force appliquée pour former la zone d'isolation.

2. Dispositif de stockage et d'extraction d'échantillon selon la revendication 1, dans lequel l'ensemble de compression comprend en outre un élément de compression, par exemple un anneau de compression, et un élément secondaire, dans lequel l'élément de compression est disposé dans le cadre de substrat, et dans lequel l'élément secondaire est disposé dans le couvercle de substrat.

3. Dispositif de stockage et d'extraction d'échantillon selon la revendication 1 ou 2, dans lequel : le cadre de substrat, le couvercle de substrat, ou les deux comprennent une ou plusieurs séparations de fente ; et/ou dans lequel le couvercle de substrat comprend un orifice d'entrée ; et/ou dans lequel le cadre de substrat et le couvercle de substrat comprennent un orifice d'entrée et un orifice de sortie respectivement; et/ou dans lequel le cadre de substrat comprend un trou correspondant à une position d'un élément de compression dans le couvercle de substrat.

4. Dispositif de stockage et d'extraction d'échantillon selon la revendication 1, dans lequel la flexion comprend une conception à double flexion.

5. Dispositif de stockage et d'extraction d'échantillon selon l'une quelconque des revendications 1 à 4, comprenant en outre un élément de chauffage en option sous la forme d'un dispositif de chauffage flexible multicouche à film mince.

6. Procédé comprenant :
la fourniture d'un dispositif de stockage et d'extraction d'échantillon selon l'une quelconque des revendications 1 à 5, incluant un cadre de substrat et un couvercle de substrat ;
la disposition d'un substrat d'échantillon dans une portion du cadre de substrat ;
la fourniture d'une zone d'isolation dans une portion du substrat d'échantillon par application d'une force de compression sur le substrat d'échantillon dans une direction déterminée ;
l'écoulement d'un fluide d'élution à travers ou sur la zone d'isolation ;
la libération de la force de compression ; et
l'analyse d'une portion du fluide d'élution écoulé hors de la zone d'isolation.

7. Procédé selon la revendication 6, dans lequel l'étape de fourniture de la zone d'isolation comprend l'isolation d'une portion du substrat d'échantillon présentant un échantillon des autres portions du substrat d'échantillon.

8. Procédé selon la revendication 6 ou 7, dans lequel l'étape de fourniture de la zone d'isolation comprend la compression des un ou plusieurs éléments d'étanchéité d'un ensemble de compression, par exemple un ensemble d'anneau de compression, contre le substrat d'échantillon.

9. Procédé selon la revendication 6, 7 ou 8, dans lequel la fourniture de la zone d'isolation comprend l'application d'une quantité déterminée de pression sur le couvercle de substrat pour former une étanchéité de compression.

10. Procédé selon la revendication 6, 7, 8 ou 9, dans lequel l'écoulement du fluide d'élution à travers ou sur la zone d'isolation comprend l'écoulement du fluide d'élution à travers ou sur la zone d'isolation en utilisant un canal fluidique disposé dans le couvercle de substrat.

11. Procédé selon la revendication 10, comprenant en outre le chauffage du fluide d'élution avant l'écoulement du fluide d'élution à travers ou sur la zone d'isolation.

12. Procédé selon l'une quelconque des revendications 6 à 11, comprenant en outre le couplage en fonctionnement du dispositif de stockage et d'extraction d'échantillon avec un dispositif externe.

13. Système comprenant :
un dispositif de stockage et d'extraction d'échantillon selon l'une quelconque des revendications 1 à 5,
dans lequel le système comprend en outre un élément chauffant couplé en fonctionnement à une unité de commande.
